# EUROPEAN PATENT SPECIFICATION

(11) **EP 0 697 170 B1**
(45) Date of publication and mention of the grant of the patent: **06.05.2004**
(21) Application number: 95305237.0
(22) Date of filing: 27.07.1995
(51) Int. Cl.: A01N 25/26, A01N 63/00, A01N 63/04

(54) **Coated pesticidal agents, processes for their preparation and compositions containing them**
Überzogene pestizide Mittel, Verfahren zu deren Herstellung sowie diese enthaltende Mittel
Agents pesticides enrobés, leurs procédés de préparation et les compositions qui les contiennent

(30) Priority: 27.07.1994 US 281916; 13.10.1994 US 322679
(43) Date of publication of application: 21.02.1996
(73) Proprietor: BASF AKTIENGESELLSCHAFT, 67056 Ludwigshafen (DE)
(72) Inventor: Fakhruddin, Ahmed, Princeton Junction, New Jersey 08550 (US)
(74) Representative: Hartz, Nikolai F., Dr.

(56) References cited:
- EP-A- 0 250 908
- WO-A-89/04170
- WO-A-89/07447
- WO-A-92/19102
- US-A- 5 124 149
- DATABASE WPI Section Ch, Week 8651, Derwent Publications Ltd., London, GB; Class A12, AN 86-335063/51 & JP-A-61 249 904 (NIPPON KAYAKU KK) 7 November 1986
- JOURNAL OF ECONOMIC ENTOMOLOGY, vol.83, no.1, February 1990, COLLEGE PARK, MARYLAND US pages 168 - 172 M.SHAPIRO ET AL 'Laboratory Evaluation of Dyes as Ultraviolet Screens for the Gypsy Moth (Lepidoptera: Lymantriidae) Nuclear Polyhedrosis Virus'

## Description

Certain pesticidal agents are inactivated by ultraviolet radiation from the sun. Because those pesticidal agents are useful for the control of undesirable pests and are applied in areas where they will be exposed to ultraviolet radiation, there is a need for photostable compositions containing those agents.

To prevent ultraviolet inactivation of pesticidal agents, compositions have been prepared which contain ultraviolet absorbers and/or reflectors and a pesticidal agent.

U.S. Patent No. 3,541,203 describes a protected virus composition for insect control. The preferred composition includes a virus, an actinic light absorbing material and a polymeric binder material. The patent discloses that to bind the admixture of an actinic light absorbing material and a virus together with an ethylcellulose polymeric material, the admixture is combined with an ethylcellulose in toluene solution. The resultant mixture is agitated, treated with polybutadiene and poured into petroleum distillate which causes the ethylcellulose to solidify to yield very small particles of ethylcellulose polymeric material having substantially homogeneously enclosed within the particles the admixture of the actinic light absorbing material and the virus. The particles are then washed several times with additional petroleum distillate to completely remove residual amounts of the liquid polybutadiene material. Unfortunat-ely, the process used to prepare the preferred compositions of U.S. Patent No. 3,541,203 is not entirely satisfactory because it requires the use of toxic materials and numerous washing steps with flammable solvents.

U.S. Patent No. 4,948,586 discloses a microencapsulated insecticidal pathogen. Four microencapsulated compositions are shown to decrease the photoinactivation of *Autographa californica* NPV. However, the microencapsulated compositions only retained from 30.7 to 71.43% of the original activity after being exposed to sunlight. U.S. Patent No. 4,948,586 discloses a method of preparing microencapsulated insecticidal pathogens which has numerous steps and is both time consuming and laborious. It is apparent that neither the process nor the microencapsulated insecticidal pathogens described in U.S. Patent No. 4,948,586 are entirely satisfactory for protecting insecticidal pathogens from the effects of ultraviolet radiation.

WO 92/19102 discloses a microencapsulated active agent such as an insecticide where the encapsulating material is lignin which also serves as sunscreen. The lignin is applied by a phase separation process.

The present invention seeks to provide a coated pesticidal agent which retains a significant amount of its original activity after exposure to ultra-violet radiation.

The present invention also seeks to provide simple, less arduous processes for the preparation of coated pesticidal agents which are more suitable for commercial manufacture.

The present invention further seeks to provide a wettable powder pesticidal composition containing a coated pesticidal agent.

### SUMMARY OF THE INVENTION

The present invention describes coated pesticidal agents which retain a significant amount of their original activity after exposure to ultraviolet radiation.

The coated pesticidal agents of the present invention comprise a pesticidal agent core surrounded by a matrix which comprises about 2 to 25% by weight of a pH-dependent polymer, 0% to about 5% by weight of a plasticizer, about 5 to 45% by weight of an ultraviolet protector, 0% to about 75% by weight of a stilbene compound, 0% to about 10% by weight of a disintegrating agent, and 0% to about 10% by weight of a glidant.

The present invention further provides processes for the preparation of coated pesticidal agents, and wettable powder pesticidal compositions comprising the coated pesticidal agents.

### BRIEF DESCRIPTION OF THE DRAWINGS

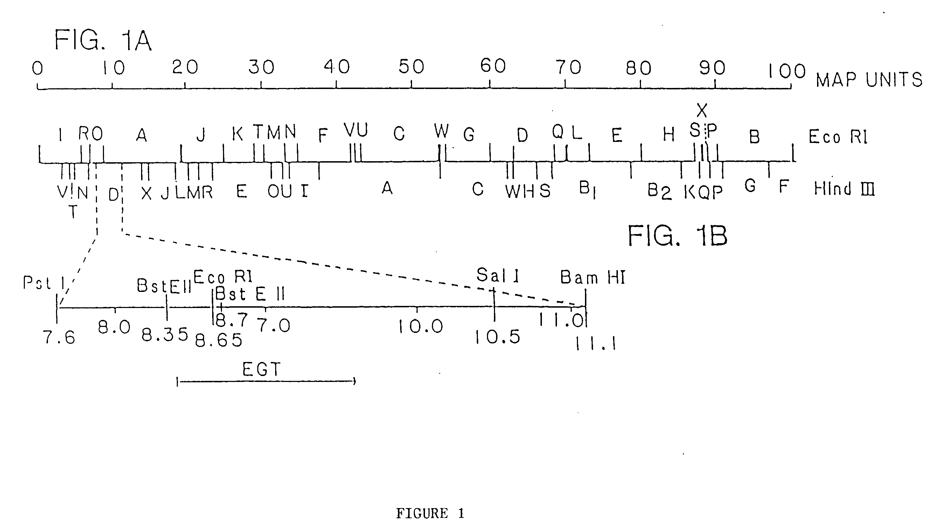

Figure 1 depicts a schematic representation of the AcMNPV genome showing the location of the egt gene. In Figure 1A the entire AcMNPV genome is presented in map units and as Eco RI and Hind III restriction maps. Figure 1B depicts a more detailed map of the region located between map units 7.6 and 11.1 and shows the location of the egt gene.

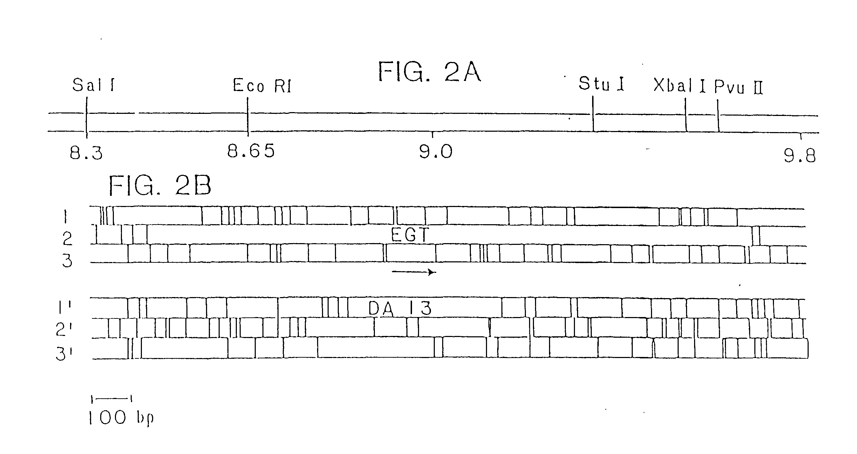

Figure 2A depicts a schematic representation of the egt gene region, which shows key restriction sites between map units 8.3 and 9.8 in the AcMNPV genome. Figure 2B depicts the organization of open reading frames in the three forward (1, 2, 3) and three reverse (1', 2', 3') reading frames of the AcMNPV genome between map units 8.3 and 9.8. The large open reading frame in frame 2 marks the position of the protein coding region of the egt gene.

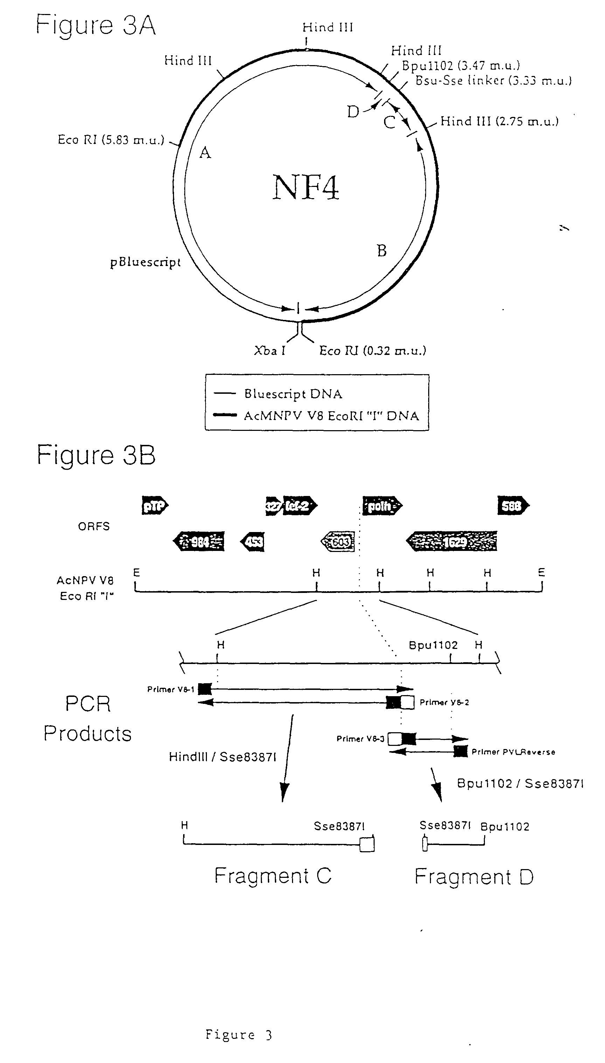

Figure 3 depicts a schematic view of the organization and derivation of the DNA fragments used to assemble the (unloaded) AcMNPV V8 transfer vector NF4. Figure 3A depicts the manner in which fragments A - D are joined to form NF4. Figure 3B depicts a schematic representation of the process used for the preparation of Fragments C and D. The arrows above the linear restriction map of the AcMNPV V8 Eco RI "I" fragment depict the location and transcriptional polarity of the major open reading frames (ORFS) located between map units 0.32 and 5.83 in the AcMNPV genome. The symbols "H" and "E" depict the positions of the recognition sites for restriction endonucleases Hind III and Eco RI, respectively.

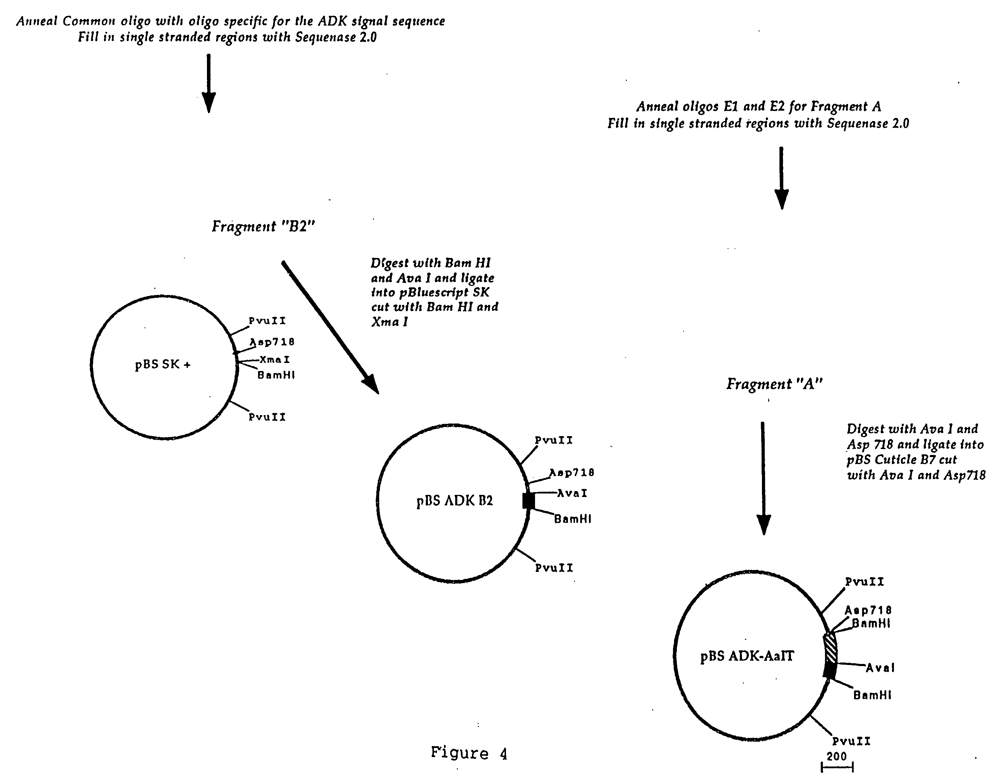

Figure 4 depicts detail of the construction of the plasmid pBS ADK-AaIT, which contains the heterologous adipokinetic hormone gene signal sequence and a codon optimized cDNA sequence encoding AaIT.

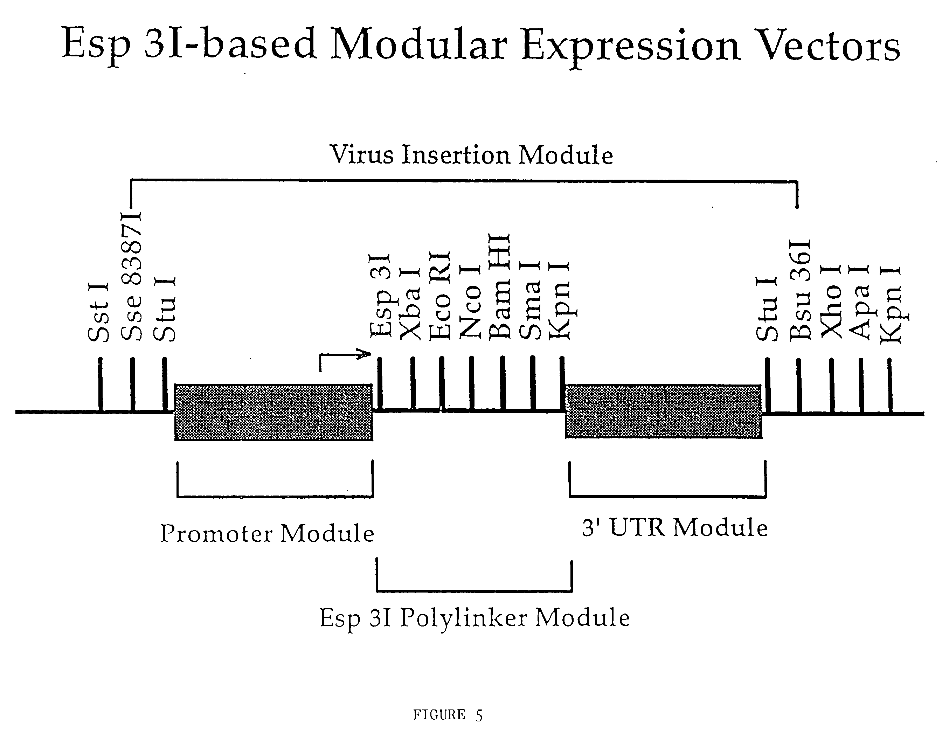

Figure 5 depicts a portion of a modular expression vector with Bsu 36I and Sse 8387I sites at opposite ends of an expression cassette containing a promoter module, a polylinker module and a 3' UTR module. The polylinker module contains an Esp 31 recognition site. The region bounded by the outermost Bsu 36I and Sse 8387I sites is defined as the virus insertion module.

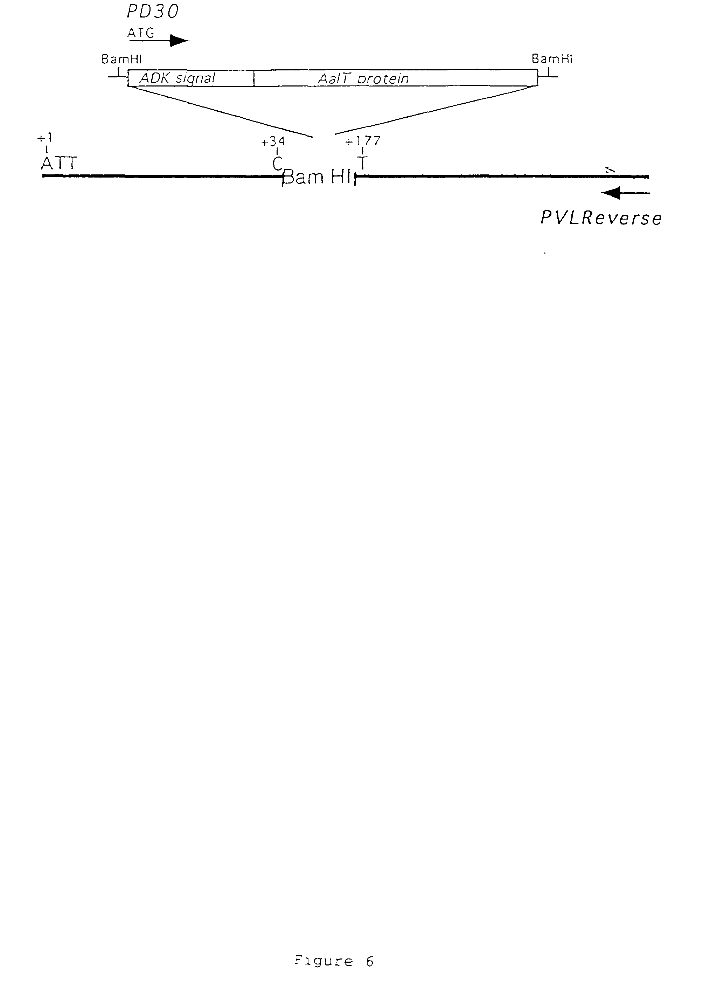

Figure 6 depicts the polymerase chain reaction (PCR) strategy for the amplification of a adipokinetic hormone gene signal/codon optimized AaIT gene, which is then digested with Bam HI.

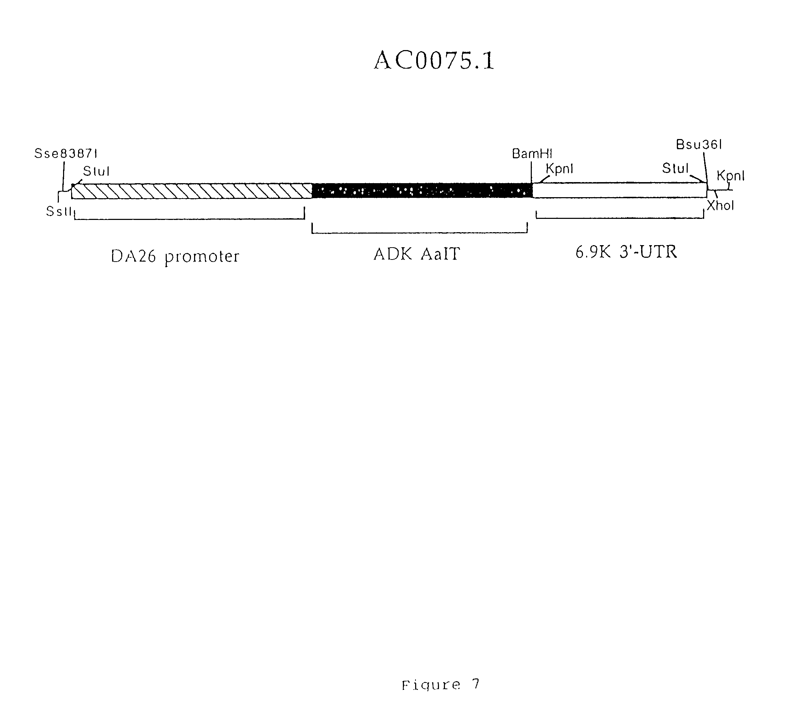

Figure 7 depicts a schematic representation of a modular expression vector (AC0075.1) formed by inserting the adipokinetic hormone gene signal/codon optimized AaIT into pMEV1.1, which contains the AcMNPV DA26 promoter.

### DETAILED DESCRIPTION OF THE INVENTION

The present invention provides processes for the preparation of coated pesticidal agents which avoid the problems associated with the methods known in the art.

One process of the present invention comprises:
a) blending a mixture of a pH-dependent polymer, a pesticidal agent, optionally a plasticizer, an ultraviolet protector, optionally a stilbene compound, optionally a disintegrating agent and optionally a glidant in an organic solvent such as acetone, C₁-C₃ alcohol, or combination thereof to produce a homogeneous suspension;
b) drying the homogeneous suspension of step (a); and optionally
c) milling the dried material of step (b).

Advantageously, the foregoing process of the present invention provides an efficient two or three step process for the preparation of coated pesticidal agents which avoids the use of multiple mixing and emulsifying steps, and avoids the use of numerous washing steps with flammable solvents. Further, the above process of this invention preferably utilizes an acetone/C₁-C₃alcohol solution which does not significantly inactivate pesticidal agents such as insecticidal pathogens. Other organic solvents taught in the art, such as methylene chloride, may significantly inactivate pesticidal agents such as insecticidal pathogens.

While the foregoing process is believed to be a significant advance over prior processes, a preferred process which avoids the use of the organic solvents has also been invented.

Uniquely, it has been found that coated pesticidal agents may be prepared by an aqueous process which comprises:
a) preparing an aqueous mixture of a pH-dependent polymer and optionally a plasticizer;
b) dissolving the pH-dependent polymer by adjusting the pH of the mixture of step (a) to a pH above the solubilization pH of the pH dependent polymer, eg to about 8.5 to 10 with a base;
c) adding a pesticidal agent, an ultraviolet protector, optionally a stilbene compound, optionally a disintegrating agent and optionally a glidant to the solution of step (b), and blending to produce a homogeneous suspension;
d) drying the homogeneous suspension of step (c); and optionally
e) milling the dried material of step (d).

Advantageously, the aqueous process of this invention does not require the use of multiple mixing and emulsifying steps, and does not require the use of numerous washing steps with flammable solvents and further does not require the use of organic solvents.

The coated pesticidal agents of the present invention preferably have a particle size less than about 20µm and more preferably have a particle size of about 2µm to 10µm. The homogeneous suspensions of this invention may be dried using any conventional drying technique. Preferably the suspensions are spray dried or air dried.

Preferred coated pesticidal agents prepared by the processes described hereinabove are those comprising a pesticidal agent core surrounded by a matrix which comprises about 2 to 25% by weight of a pH-dependent polymer, up to about 5% by weight of a plasticizer, about 5 to 45% by weight of an ultraviolet protector, up to about 75% by weight of a stilbene compound, up to about 10% by weight of a disintegrating agent, and up to about 10% by weight of a glidant.

The ratio of the pesticidal agent to the matrix is preferably about 1:1 to 1:10. And the ratio of acetone to the C₁-C₃alcohol is preferably about 1:9 to 9:1 and more preferably about 1:4 to 2:3. C₁-C₃alcohols suitable for use in the organic process include methanol, ethanol, isopropanol and n-propanol with isopropanol being preferred.

Pesticidal agents suitable for use in the present invention include chemical and biological insecticides, acaricides, nematicides and fungicides or mixtures thereof which are inactivated by ultraviolet radiation. Preferred pesticidal agents are insecticidal pathogens such as viral pathogens, bacterial pathogens and fungal pathogens. Viral pathogens suitable for use include wild gypsy moth NPV; *Autographa californica* NPV's such as AcMNPV E2, AcMNPV L1, AcMNPV V8, V8vEGTDEL and V8vEGTDEL-AaIT; Douglas fir tossock moth NPV; European pine saw fly NPV; and *Heliothis zea* NPV. Most preferred pesticidal agents for use in this invention are wild gypsy moth NPV, AcMNPV E2, AcMNPV L1, AcMNPV V8, V8vEGTDEL, V8vEGTDEL-AaIT, and *Heliothis zea* NPV.

AcMNPV E2 is described in EP 621337 published October 26,1994

pH-Dependent polymers suitable for use in the present invention include polymers which are essentially insoluble below about pH 5 such as methacrylic acid and methyl methacrylate copolymers, maleic anhydride and styrene copolymers and the like or mixtures thereof. Preferred pH-dependent polymers include methacrylic acid and methyl methacrylate copolymers, methacrylic acid and methyl methacrylate copolymer mixtures and maleic anhydride and styrene copolymers. Most preferred pH-dependent polymers are Eudragit®S (methacrylic acid and methyl methacrylate copolymer wherein the ratio of free carboxyl groups to esters is about 1:2; Röhm Pharma GmbH, Weiterstadt, Germany), Eudragit®L (methacrylic acid and methyl methacrylate copolymer wherein the ratio of free carboxyl groups to esters is about 1:1; Röhm Pharma GmbH) and mixtures thereof.

Plasticizers suitable for use in the present invention include any of the conventional agents known in the art such as polyethylene glycols, polypropylene glycols, diethyl phthalate, dibutyl phthalate, citric acid esters, castor oil, triacetin and the like or mixtures thereof with polyethylene glycols having a molecular weight of about 300 to 1,000 being preferred.

Ultraviolet protectors are used in the present invention to reduce the photoinactivation of the pesticidal agent. Ultraviolet protectors suitable for use include ultraviolet absorbers and ultraviolet reflectors or mixtures thereof. Ultraviolet absorbers include various forms of carbon such as carbon black; benzophenones such as 2-hydroxy-4-methoxybenzophenone (CYASORB®UV 9, Cytec Ind.), 2,2'-dihydroxy-4-methoxybenzophenone (CYASORB®UV 24, Cytec Ind.), 2-hydroxy-4-acryloyloxyethoxybenzophenone (CYASORB®UV 2098, Cytec Ind.), 2-hydroxy-4-n-octoxybenzophenone (CYASORB®UV 531, Cytec Ind.) and the like; and dyes such as congo red, malachite green, malachite green hydrochloride, methyl orange, methyl green, brilliant green, acridine yellow, FDC green, FDC yellow, FDC red and the like. Ultraviolet reflectors include titanium dioxide and the like. Preferred ultraviolet protectors include carbon black, benzophenones, dyes and titanium dioxide with carbon black, CYASORB®UV 9 and CYASORB®UV 24 being most preferred.

Bases suitable for use in the aqueous process of this invention include ammonium hydroxide, alkali metal hydroxides, alkaline earth metal hydroxides and the like with ammonium hydroxide being preferred. Certain insecticidal viral pathogens may be deactivated at a pH greater than 10. Therefore it is preferred to select an amount of a base which will adjust the pH to about 8.5 to 10 to ensure ready solubilization and lessen the chance of deactivation.

Stilbene compounds are used in this invention to enhance pesticidal activity of the pesticidal agent. Stilbene compounds suitable for use in this invention are described in U.S. Patent No. 5,246,936.

Preferred stilbene compounds are the analogues of 4,4'-diamino-2,2'-stilbene disulfonic acid, namely, a Calcofluor White (available from Sigma Chemical Co., St. Louis, Mo.) such as Calcofluor White M2R, Calcofluor White ABT, Calcofluor White LD, Calcofluor White RWP, etc.; a Blancophor (available from Mobay Chemicals, Pittsburgh, Pa.) such as Blancophor BBH, Blancophor MBBH, Blancophor BHC, etc.; an INTRAWITE® (a heterocyclic stilbene derivative, available from Crompton and Knowles Corp., Charlotte, N.C.) such as INTRAWITE®CF, etc.; a Leucophor (available from Sandoz Chemicals Corp., Charlotte, N.C.) such as Leucophor BS, Leucophor BSB, Leucophor EKB, Leucophor PAB, etc.; a Phorwite (available from Mobay Chemicals, Pittsburgh, Pa.) such as Phorwite AR, Phorwite BBU, Phorwite BKL, Phorwite CL, Phorwite RKK, etc. and the like. Blancophor BBH, Calcofluor White M2R and Phorwite AR are the most preferred stilbene compounds.

Disintegrating agents are used in the present invention to shorten the milling time and enhance the particle size reduction of the dried material. Disintegrating agents suitable for use in this invention include salts of the condensation products of formaldehyde with the sulfonation products of polycyclic aromatic compounds, hydrophilic starches such as dextran, carboxy methylcellulose, polyvinyl pyrrolidine and the like or mixtures thereof. Preferred disintegrating agents are salts of the condensation products of formaldehyde with the sulfonation products of polycyclic aromatic compounds such as salts of the condensation products of formaldehyde with naphthalene sulfonates, petroleum sulfonates and lignin sulfonates with the sodium sulfonate of naphthalene formaldehyde condensates such as MORWET®D425 (Witco, Houston, Texas), LOMAR®PW (Henkel, Cincinnati, Ohio) and DARVAN®1 (R.T. Vanderbilt Co., Norwalk, Connecticut) being most preferred.

Glidants are used in the processes of this invention to keep the dried, coated pesticidal agents from sticking together. Glidants suitable for use in this invention include talc, magnesium stearate, calcium stearate, calcium sulfate and the like or mixtures thereof with talc being preferred.

Other additives such as preservatives, stabilizers (trehalose), anti-mold agents, anti-fungal agents, anti-bacterial agents and the like may also be included in the matrix of the present invention. Clearly, anti-fungal agents and anti-bacterial agents generally would not be used when fungal pathogens and bacterial pathogens, respectively, are coated.

Preferred coated pesticidal agents of the present invention are those comprising a pesticidal agent core surrounded by a matrix which comprises about 2 to 20% by weight of a pH-dependent polymer, up to about 3% by weight of a plasticizer, about 5 to 35% by weight of an ultraviolet protector, about 25 to 75% by weight of a stilbene compound, up to about 10% by weight of a disintegrating agent, and up to about 10% by weight of a glidant.

The present invention also provides a wettable powder pesticidal composition which comprises about 2 to 25% by weight of a wetting agent; about 2 to 40% by weight of a dispersing agent; about 10 to 70% by weight of a bulking agent; about 1 to 10% by weight of a flow enhancing agent; up to about 20% by weight of a pH-modifying agent; and about 5 to 75% by weight of a coated pesticidal agent which comprises a pesticidal agent core surrounded by a matrix which comprises about 2 to 25% by weight of a pH-dependent polymer, up to about 5% by weight of a plasticizer, about 5 to 45% by weight of an ultraviolet protector, up to about 75% by weight of a stilbene compound, up to about 10% by weight of a disintegrating agent, and up to about 10% by weight of a glidant.

Preferred wettable powder pesticidal compositions of the present invention are those comprising about 2 to 15% by weight of a wetting agent; about 2 to 15% by weight of a dispersing agent; about 10 to 60% by weight of a bulking agent; about 1 to 5% by weight of a flow enhancing agent; up to about 20% by weight of a pH-modifying agent; and about 5 to 75% by weight of a coated pesticidal agent which comprises a pesticidal agent core surrounded by a matrix which comprises about 2 to 25% by weight of a pH-dependent polymer, up to about 5% by weight of a plasticizer, about 5 to 45% by weight of an ultraviolet protector, up to about 75% by weight of a stilbene compound, up to about 10% by weight of a disintegrating agent, and up to about 10% by weight of a glidant.

Wetting agents suitable for use in the present invention include any of the conventional agents known in the art. Preferred wetting agents include anionic agents such as sodium N-methyl-N-oleoyltaurate, octylphenoxy polyethoxy ethanol, nonylphenoxy polyethoxy ethanol, sodium dioctyl sulfosuccinate, sodium dodecyl benzene sulfonate, sodium lauryl sulfate, sodium alkyl naphthalene sulfonate, sodium sulfonated alkyl carboxylate and the like or mixtures thereof. A mixture of sodium alkyl naphthalene sulfonate and sodium sulfonated alkyl carboxylate (MORWET®EFW, Witco) is the most preferred wetting agent.

Dispersing agents useful in the wettable powder pesticidal compositions of this invention include any of the conventional agents known in the art. Preferred dispersing agents are anionic agents such as salts of the condensation products of formaldehyde with the sulfonation products of polycyclic aromatic compounds, sodium lignosulfonate and the like or mixtures thereof with the sodium sulfonate of naphthalene formaldehyde condensates such as MORWET®D425 (Witco), LOMAR®PW (Henkel) and DARVAN ®1 (R.T. Vanderbilt Co.) being most preferred.

Bulking agents suitable for use in the compositions of the present invention include natural and synthetic clays and silicates, for example: natural silicas such as diatomaceous earths; magnesium silicates such as talcs; magnesium aluminum silicates such as attapulgites and vermiculites; aluminum silicates such as kaolinites, montmorillonites and micas; and hydrated aluminum silicates such as kaolin clay. Preferred bulking agents are hydrated aluminum silicates, aluminum silicates, magnesium silicates and magnesium aluminum silicates with kaolin clay being the most preferred bulking agent. Flow enhancing agents useful in the wettable powder pesticidal compositions of this invention are conventional flow enhancing agents known in the art with silicates such as calcium silicates being preferred.

pH-Modifying agents are used to maintain the pH of aqueous tank-mixes prepared from the compositions of this invention below about pH 5. pH-Modifying agents suitable for use include potassium hydrogen phthalate and organic acids with citric acid being preferred.

The wettable powder pesticidal compositions of the present invention may be prepared by blending a mixture of a wetting agent, a dispersing agent, a bulking agent, a flow enhancing agent and optionally a pH-modifying agent to form a premix. The premix is then blended with a coated pesticidal agent to form the desired wettable powder pesticidal composition of the present invention.

For the control of pests, the wettable powder pesticidal compositions of this invention are diluted with water to form an aqueous tank-mix and the tank-mix is applied directly to the pests, their breeding grounds, food supply or habitat.

Other ingredients such as attractants, stickers, anti-foaming agents and the like may be added to the wettable powder compositions of this invention. However, those additional ingredients are generally added separately to the tank-mix. An adjuvant or mixture of adjuvants may also be added to the tank-mix.

In order to facilitate a further understanding of the invention, the following examples are presented primarily for the purpose of illustrating more specific details thereof. The invention should not be deemed limited thereby except as defined in the claims.

Unless otherwise noted, standard molecular biological techniques are utilized according to the protocols described in Sambrook et al. Molecular Cloning: A Laboratory Manual, 2nd ed., Cold Spring Harbor Press, Cold Spring Harbor, N.Y. (1989). Standard techniques for baculovirus growth and production are utilized according to the protocols described in Summers and Smith A Manual Of Methods For Baculovirus Vectors and Insect Cell Culture Procedures, Dept. of Entomology, Texas Agricultural Experimental Station and Texas A & M University, College Station, Texas 77843-2475, Texas Agricultural Experiment Station Bulletin No. 1555 (1987).

A deposit of AcMNPV V8 has been made under the Budapest Treaty and given ATCC No VR-2465.

### EXAMPLE 1

### Preparation of coated V8vEGTDEL polyhedrin inclusion bodies - Aqueous Process

Ammonium hydroxide solution (28% NH₃) is added to a mixture of Eudragit®S100 (62 g, Röhm Pharma Co.) and PEG 400 (6.2 g, poly(ethylene glycol) average M.W. 400, Aldrich Chemical Co.) in deionized water (551.8 g) until a pH of about 9.4 is obtained. The resulting mixture is stirred for 30 minutes to obtain a solution. V8vEGTDEL polyhedrin inclusion bodies (62 g, average size about 2 µm, about 10¹¹ bodies per gram), Blancophor BBH (248 g, stilbene brightner, Miles Inc.) and CYASORB®UV 9 (31 g, average particle size about 2 µm, Cytec Ind.) are added to the pH adjusted aqueous solution. The resultant mixture is stirred for 30 minutes and air dried with blending to obtain solid granules. The solid granules are air milled to give coated V8vEGTDEL polyhedrin inclusion bodies (362 g, average size about 5 µm). The coated V8vEGTDEL polyhedrin inclusion bodies prepared above are identified as composition 1 in Table II.

Using essentially the same procedure, but using the ingredients listed in Table I, the coated pesticidal agents identified as compositions 2-10 in Table II are prepared.

**TABLE I**

| Pesticidal Agent | |
|---|---|
| a. | V8vEGTDEL polyhedrin inclusion bodies |
| b. | *Heliothis zea* NPV |
| c. | AcMNPV |
| d. | 1:1 mixture of *Heliothis zea* NPV and AcMNPV |

| pH-Dependent Polymer | |
|---|---|
| e. | Eudragit®S100 |

| Plasticizer | |
|---|---|
| f. | PEG 400 |

| UV-Protector | |
|---|---|
| g. | CYASORB®UV 9 |
| h. | Charcoal |

| Stilbene Compound | |
|---|---|
| i. | Blancophor BBH |

| Glidant | |
|---|---|
| j. | Talc |

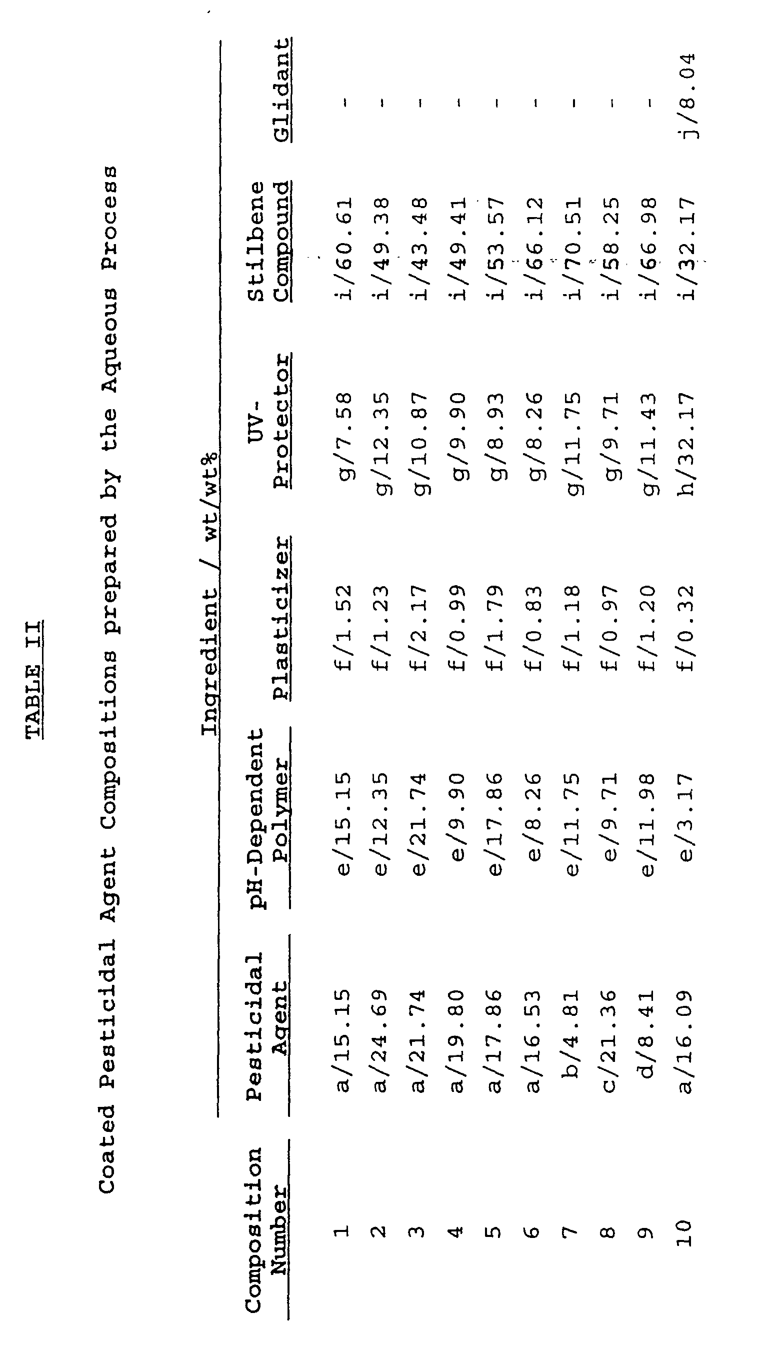

### EXAMPLE 2

### Preparation of coated V8vEGTDEL polyhedrin inclusion bodies - Organic Process

V8vEGTDEL polyhedrin inclusion bodies (43.24 g, average size about 2 µm, about 10¹¹ bodies per gram), Blancophor BBH (86.44g) and CYASORB®UV 9 (31.93 g) are added to a solution of Eudragit®S100 (5.88 g) and PEG 400 (1.51 g) in a 30:70 acetone/isopropanol solution (195.15 g). The resultant mixture is stirred for several minutes and air dried with blending to obtain solid granules. The solid granules are milled through a 60 mesh screen to give coated V8vEGTDEL polyhedrin inclusion bodies having an average size of about 10 µm. The coated V8vEGTDEL polyhedrin inclusion bodies prepared above are identified as composition 11 in Table IV.

Using essentially the same procedure, but using the ingredients listed in Table III, the coated pesticidal agents identified as compositions 12-28 in Table IV are prepared.

**TABLE III**

| Pesticidal Agent | |
|---|---|
| a. | V8vEGTDEL polyhedrin inclusion bodies |
| b. | Wild gypsy moth NPV |

| pH-Dependent Polymer | |
|---|---|
| c . | Eudragit®S100 |
| d. | Cypress®48 (maleic anhydride/styrene copolymer, |
| | Cytec Ind.) |

| Plasticizer | |
|---|---|
| e. | PEG 400 |

| UV-Protector | |
|---|---|
| f. | CYASORB®UV 9 |
| g. | Charcoal |
| h. | TiO₂ |

| Stilbene Compound | |
|---|---|
| i. | Blancophor BBH |

| Disintegrating Agent | |
|---|---|
| j. | MORWET®D425 |

| Stabilizer | |
|---|---|
| k. | Trehalose |

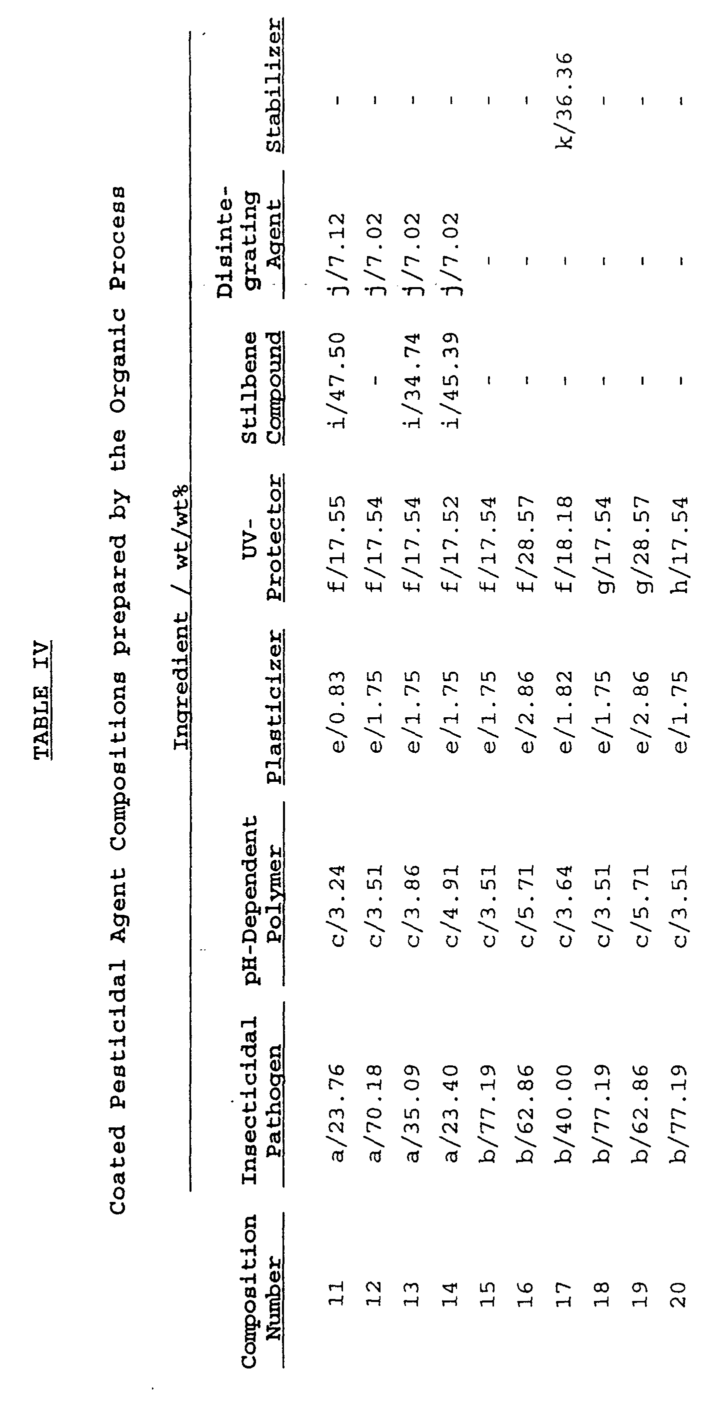

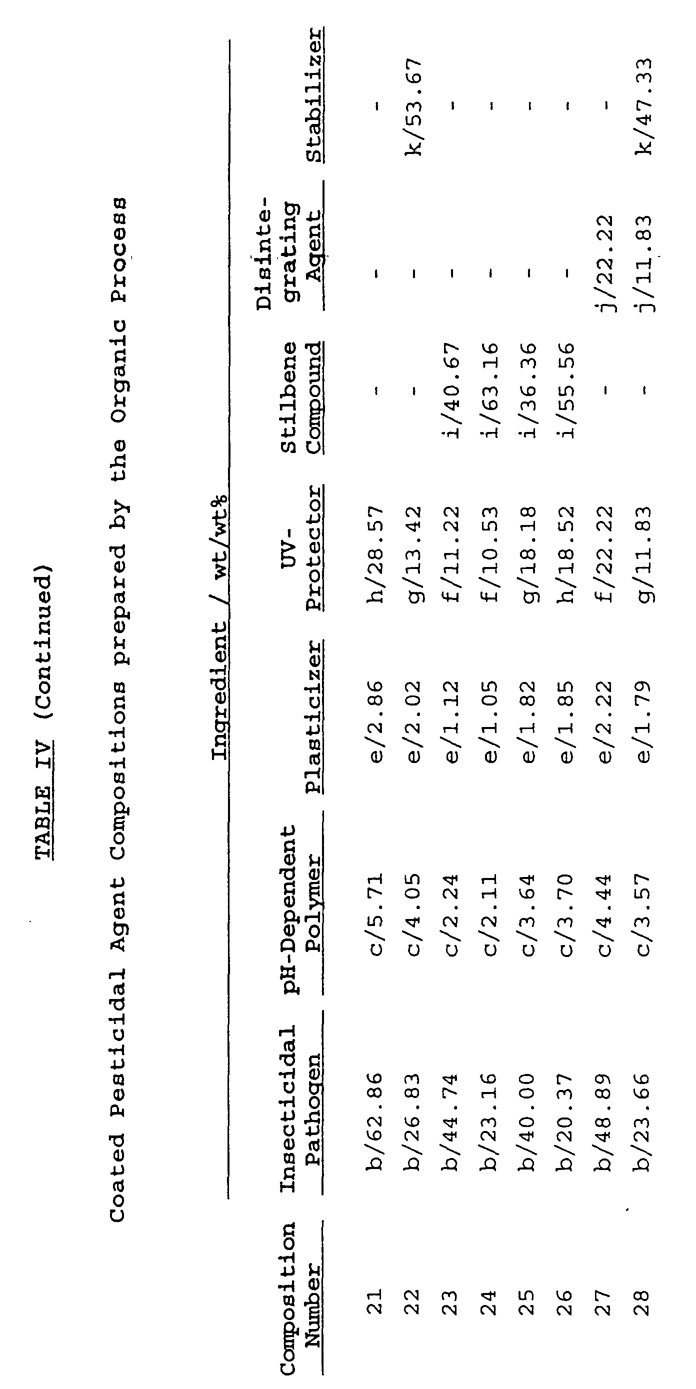

### EXAMPLE 3

### Preparation of wettable powder pesticidal compositions

The coated V8vEGTDEL polyhedrin inclusion bodies identified as composition 1 in Table II (362 g) are added to a premix of MORWET®EFW (13.1 g), MORWET®D425 (26.2 g), kaolin clay (91.6 g), synthetic calcium sulfate (6.5 g, MICRO-CEL®E, Manville Co.) and citric acid (0.7 g). The resultant mixture is blended to obtain the wettable powder composition identified as composition 29 in Table V.

Using essentially the same procedure, the wettable powder compositions identified as compositions 30-53 in Table V are prepared.

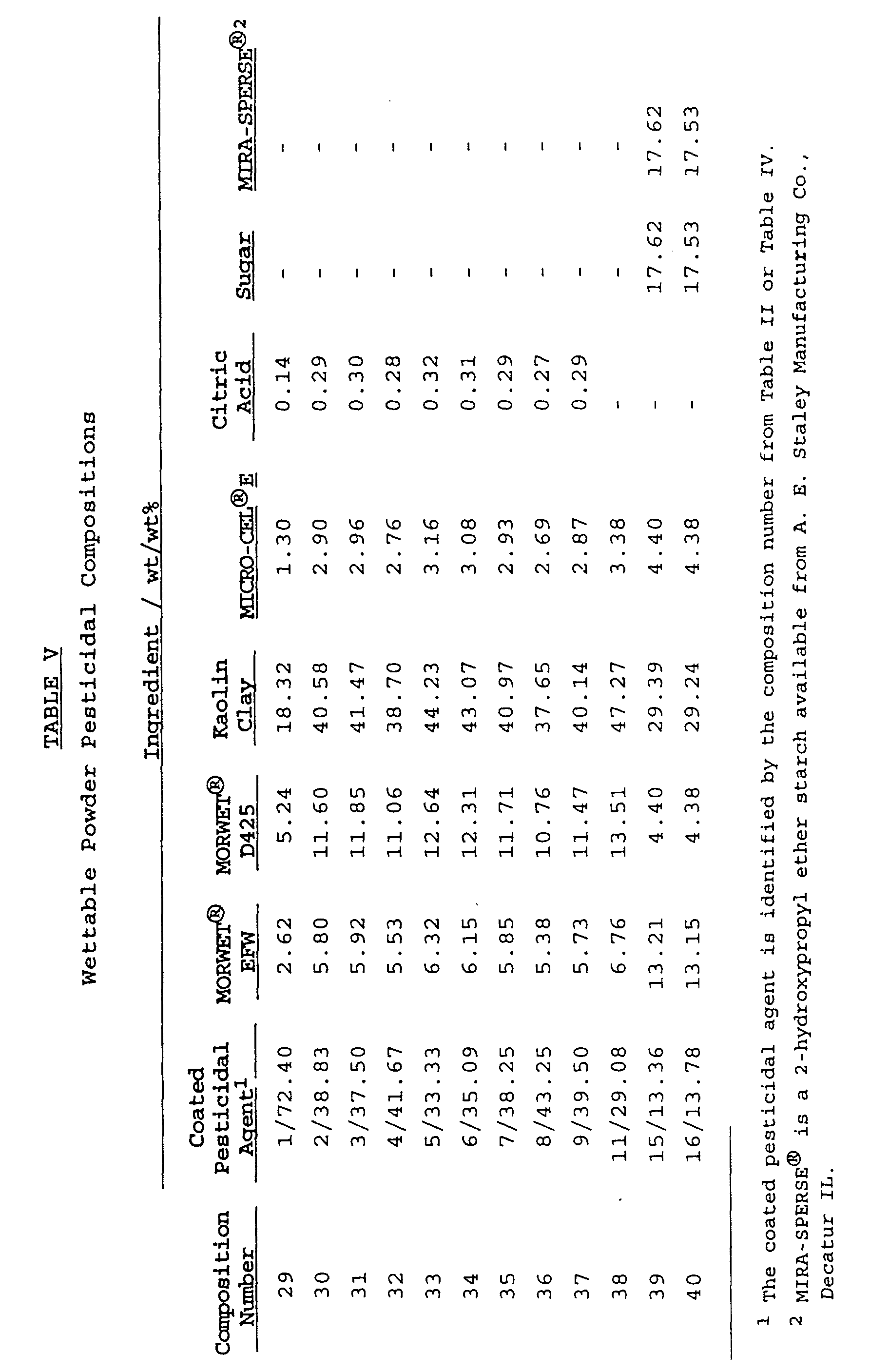

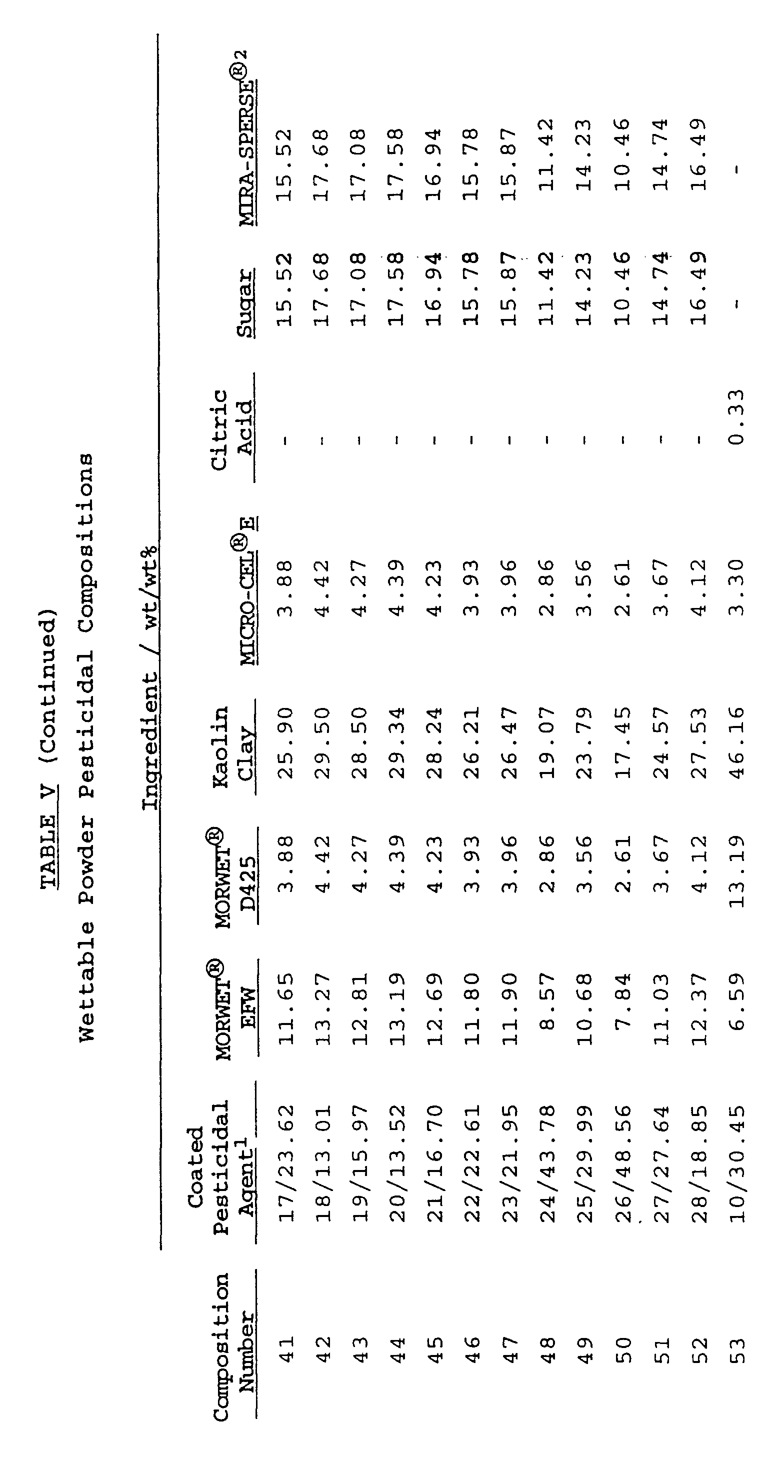

### EXAMPLE 4

### Insecticidal evaluations of non-irradiated and irradiated wettable powder pesticidal compositions against L. dispar

Wettable powder insecticidal pathogen compositions are suspended in distilled water and diluted to a concentration of 2.4 x 10⁵ coated insecticidal polyhedrin inclusion bodies per mL. The resultant suspension (0.5 mL) is pipetted to the surface of wheat germ diet in a 180 mL plastic cup. Each cup is exposed to ultraviolet radiation (one Westinghouse BLB bulb and one Phillips F40 UVB bulb set three inches apart, the distance from the center line of the radiation source and the diet surface is 4 inches) for either 0 or 80 minutes. Ten second instar *L*. *dispar* caterpillars are placed in each cup. The cups are covered and maintained in darkness at 29°C, 55-60% relative humidity. After 13 days, the cups are examined and mortality by virus infection is determined.

The results are summarized in Table VI wherein the effectiveness of each composition is expressed as the percent of original activity remaining after ultraviolet exposure (% OAR), i.e. % mortality caused by irradiated composition ö % mortality caused by non-irradiated composition x 100. The control composition used in the evaluations is identified below.

| **Control Composition** | |
|---|---|
| **Ingredient** | **wt/wt%** |
| ¹Coated insecticidal pathogen | 11.19 |
| MORWET®EFW | 13.54 |
| MORWET®D425 | 4.51 |
| Kaolin Clay | 30.12 |
| MICRO-CEL®E | 4.51 |
| Sugar | 18.06 |
| MIRA-SPERSE® | 18.06 |

| | |
|---|---|
| ¹ 93.62 wt/wt% wild gypsy moth NPV, 4.26% wt/wt% Eudragit® S100, and 2.13 wt/wt% PEG 400. | |

**TABLE VI**

| **Insecticidal Evaluations Against *L. dispar*** | | | |
|---|---|---|---|
| | **% Mortality** | | |
| C**omposition Number**^{**1**} | **Irradiated** | **Non-Irradiated** | **% OAR**^{**2**} |
| Control | 47.5 | 100.0 | 47.5 |
| 39 | 70.0 | 100.0 | 70.0 |
| 40 | 76.7 | 99.2 | 77.2 |
| 41 | 87.5 | 99.2 | 88.2 |
| 42 | 91.7 | 98.3 | 93.2 |
| 43 | 98.3 | 99.2 | 99.2 |
| 44 | 57.5 | 100.0 | 57.5 |
| 45 | 65.0 | 96.7 | 67.3 |
| 46 | 82.5 | 98.3 | 84.0 |
| 47 | 91.7 | 98.3 | 93.2 |
| 48 | 90.0 | 99.2 | 90.8 |
| 49 | 90.0 | 98.3 | 91.6 |
| 50 | 87.9 | 100.0 | 87.9 |
| 51 | 77.5 | 99.2 | 78.2 |
| 52 | 90.0 | 99.2 | 90.7 |

| | | | |
|---|---|---|---|
| ¹ Composition number from Table V. | | | |
| ² Percentage of original activity remaining after ultraviolet exposure. | | | |

### EXAMPLE 5

### Insecticidal evaluations of wettable powder pesticidal compositions against Helicoverpa zea

Plastic bioassay trays containing 32 open-faced wells (4 x 4 x 2.5 cm, L x W x H, C-D International, Inc. ) per tray are utilized as test arenas in this evaluation. Five mL of Stoneville diet (soybean/wheat germ) is poured into each tray-well and allowed to harden. Aqueous suspensions (0.4 mL) of wettable powder insecticidal pathogen compositions are evenly spread over the surface of the hardened diet to provide from 4 x 10⁵ to 4 x 10⁷ V8vEGTDEL coated or uncoated polyhedrin inclusion bodies per well. After drying the trays in a laminar flow hood, one three-day-old *Helicoverpa zea* larvae is placed on the surface of the diet in each tray-well. The wells are covered with an adhesive, vented clear plastic sheet (C-D International, Inc.), held under constant fluorescent light and at a temperature of about 27°C. Five days and ten days after treatment, the wells are examined and larval mortality measurements are made.

The results are summarized in Table VII. The control compositions used in the evaluations are identified below.

| **Control Composition** | **Ingredient** | **wt/wt%** |
|---|---|---|
| A | Uncoated V8vEGTDEL polyhedrin inclusion bodies | 10.00 |
| | MORWET®EFW | 8.53 |
| | MORWET®D425 | 17.06 |
| | Kaolin Clay | 59.71 |
| | MICRO-CEL®E | 4.27 |
| | Citric Acid | 0.43 |
| | | |
| B | Uncoated V8vEGTDEL polyhedrin inclusion bodies | 8.69 |
| | MORWET®EFW | 8.71 |
| | MORWET®D425 | 17.40 |
| | Kaolin Clay | 60.86 |
| | MICRO-CEL®E | 4.35 |

**TABLE VII**

| **Insecticidal Evaluations Against *Helicoverpa zea*** | | | |
|---|---|---|---|
| **Composition** | **Concentration** | **% Mortality** | |
| **Number**^{**1**} | **(bodies/well)** | **5 Days** | **10 Days** |
| Control A | 4 x 10⁷ | 49 | 91 |
| | 4 x 10⁶ | 47 | 93 |
| | 4 x 10⁵ | 29 | 63 |
| | | | |
| Control B | 4 x 10⁷ | 33 | 75 |
| | 4 x 10⁶ | 35 | 70 |
| | 4 x 10⁵ | 18 | 47 |
| | | | |
| 29 | 4 x 10⁷ | 77 | 100 |
| | 4 x 10⁶ | 58 | 100 |
| | 4 x 10⁵ | 35 | 77 |
| | | | |
| 38 | 4 x 10⁷ | 43 | 95 |
| | 4 x 10⁶ | 45 | 80 |
| | 4 x 10⁵ | 25 | 56 |

| | | | |
|---|---|---|---|
| ¹ Composition number from Table V. | | | |

### EXAMPLE 6

### Evaluation of non-irradiated and irradiated wettable powder compositions against H. zea and H. virescens

Plastic bioassay trays containing 32 open-faced wells (4 x 4 x 2.5 cm, L x W x H, C-D International, Inc.) per tray are utilized as test arenas in this evaluation. Five mL of Stoneville diet (soybean/wheat germ) is poured into each tray-well and allowed to harden. Aqueous suspensions (0.4 mL) of the wettable powder insecticidal pathogen compositions are evenly spread over the surface of the hardened diet to provide 4 x 10⁶ V8vEGTDEL coated or uncoated polyhedrin inclusion bodies per well. Some of the treated trays are then held under ultraviolet lamps (two FS4OUVB bulbs set 30 cm above the trays, Phillips Co.) for either one or two hours. Trays selected for two hours of irradiation are provided with an additional 0.4 mL of deionized water per well at the one hour time interval to prevent the diet from over-drying and cracking. All trays are then infested with a single three-day-old *H. zea* or four-day-old *H. virescens* larvae. The wells are covered with an adhesive, vented clear plastic sheet (C-D International, Inc.), held under constant fluorescent light and at a temperature of about 27°C. Ten days after treatment, the wells are examined and larval mortality measurements are made.

The results are summarized in Table VIII. The control composition used in the evaluations is identified below.

| **Control Composition** | |
|---|---|
| **Ingredient** | **wt/wt%** |
| Uncoated V8vEGTDEL polyhedrin inclusion bodies | 4.93 |
| MORWET®EFW | 9.01 |
| MORWET®D425 | 18.02 |
| Kaolin Clay | 63.09 |
| MICRO-CEL®E | 4.51 |
| Citric Acid | 0.45 |

**TABLE VIII**

| **Evaluation of non-irradiated and irradiated wettable powder compositions against *H. zea* and *H. virescens*** | | | |
|---|---|---|---|
| **Composition** | **Irradiation Exposure** | **Mean % Larval Mortality** | |
| **Number** | **(hours)** | ***H. zea*** | **H. virescens** |
| Control | 0 | 59 | 77 |
| | 1 | 46 | 56 |
| | 2 | 40 | 36 |
| 53¹ | 0 | 91 | 89 |
| | 1 | 90 | 87 |
| | 2 | 83 | 56 |

| | | | |
|---|---|---|---|
| ¹ Composition number from Table V. | | | |

### EXAMPLE 7

### Solvent compatibility evaluations

The following evaluation is used to determine the effect of various solvents and mixtures thereof on the activity of *Autographa californica* polyhedrin inclusion bodies. A mixture of *Autographa californica* polyhedrin inclusion bodies (0.55 g) and the appropriate solvent or solvent mixture (1.5 mL) is held in a conical tube for 10 or 60 minutes. The tubes are then centrifuged and the supernatant is decanted. The solids are dried under vacuum in a dessicator. The dried solids are then evaluated against *Heliothis virescens* according to the procedure described in Example 6 (no irradiation).

The results are summarized in Table IX. As can be seen from the data in Table IX, the *Autographa californica* polyhedrin inclusion bodies mixed with methylene chloride for 10 and 60 minutes are significantly less active against *Heliothis virescens* than the bodies mixed with acetone, isopropanol and a 30:70 acetone/isopropanol mixture.

**TABLE IX**

| **Solvent Compatibility Evaluations** | | | |
|---|---|---|---|
| **Solvent** | **Time held (minutes)** | **Rate (bodies/well)** | **% Mortality** |
| Acetone | 10 | 4 x 10² | 19 |
| | 60 | 4 x 10² | 25 |
| | 10 | 4 x 10⁴ | 100 |
| | 60 | 4 x 10⁴ | 100 |
| | | | |
| Isopropanol | 10 | 4 x 10² | 31 |
| | 60 | 4 x 10² | 13 |
| | 10 | 4 x 10⁴ | 100 |
| | 60 | 4 x 10⁴ | 100 |
| | | | |
| Acetone/Isopropanol (30:70) | 10 | 4 x 10² | 34 |
| | 60 | 4 x 10² | 19 |
| | 10 | 4 x 10⁴ | 100 |
| | 60 | 4 x 10⁴ | 100 |
| | | | |
| Methylene chloride | 10 | 4 x 10² | 9 |
| | 60 | 4 x 10² | 6 |
| | 10 | 4 x 10⁴ | 84 |
| | 60 | 4 x 10⁴ | 19 |

## Claims

1. A process for the preparation of a coated pesticidal agent which comprises:
a) preparing an aqueous mixture of a pH-dependent polymer and optionally a plasticizer;
b) dissolving the pH-dependent polymer by adjusting the pH of the mixture of step (a) to a pH above the solubilization pH of the pH dependent polymer;
c) adding a pesticidal agent, an ultraviolet protector, optionally a stilbene compound, optionally a disintegrating agent and optionally a glidant to the solution of step (b), and blending to produce a homogeneous suspension;
d) drying the homogeneous suspension of step (c); and optionally
e) milling the dried material of step (d).

2. The process according to claim 1 wherein the pH-dependent polymer is selected from the group consisting of a methacrylic acid and methyl methacrylate copolymer, a mixture of methacrylic acid and methyl methacrylate copolymers and a maleic anhydride and styrene copolymer; the plasticizer is selected from the group consisting of a polyethylene glycol, a polypropylene glycol, diethyl phthalate, dibutyl phthalate, a citric acid ester, castor oil and triacetin; the base is selected from the group consisting of ammonium hydroxide, an alkali metal hydroxide and an alkaline earth metal hydroxide; the pesticidal agent is an insecticidal pathogen; the ultraviolet protector is selected from the group consisting of carbon black, a benzophenone, a dye and titanium dioxide; the pH is adjusted in step (b) to pH 8.5 to 10; the disintegrating agent is selected from the group consisting of salts of the condensation products of formaldehyde with the sulfonation products of polycyclic aromatic compounds, a hydrophilic starch, carboxy methylcellulose and polyvinyl pyrrolidine; and the glidant is selected from the group consisting of talc, magnesium stearate, calcium stearate and calcium sulfate.

3. The process according to claim 2 wherein the plasticizer is a polyethylene glycol having a molecular weight of about 300 to 1,000; the base is ammonium hydroxide; the insecticidal pathogen is a virus selected from the group consisting of wild gypsy moth NPV, AcMNPV E2, AcMNPV L1, AcMNPV V8, V8vEGTDEL, V8vEGTDEL-AalT and *Heliothis zea* NPV; the disintegrating agent is a sodium sulfonate of a naphthalene formaldehyde condensate; and the glidant is talc and wherein the coated pesticidal agent has a particle size less than about 20 µm.

4. A process for the preparation of a coated pesticidal agent which comprises:
a) blending a mixture of a pH-dependent polymer, a pesticidal agent, optionally a plasticizer, an ultraviolet protector, optionally a stilbene compound, optionally a disintegrating agent and optionally a glidant in an organic solvent selected from the group consisting of acetone, a C₁-C₃ alcohol, and mixtures thereof;
b) drying the homogeneous suspension of step (a); and optionally
c) milling the dried material of step (b).

5. The process according to claim 4 wherein the organic solvent is a mixture of acetone and a C₁-C₃ alcohol and the ratio of acetone to the C₁-C₃ alcohol is about 1:9 to 9:1.

6. The process according to claim 4 wherein the pH-dependent polymer is selected from the group consisting of a methacrylic acid and methyl methacrylate copolymer, a mixture of methacrylic acid and methyl methacrylate copolymers and a maleic anhydride and styrene copolymer; the pesticidal agent is an insecticidal pathogen; the plasticizer is selected from the group consisting of a polyethylene glycol, a polypropylene glycol, diethyl phthalate, dibutyl phthalate, a citric acid ester, castor oil and triacetin; the ultraviolet protector is selected from the group consisting of carbon black, a benzophenone, a dye and titanium dioxide; the disintegrating agent is selected from the group consisting of salts of the condensation products of formaldehyde with the sulfonation products of polycyclic aromatic compounds, a hydrophilic starch, carboxymethyl cellulose and polyvinyl pyrrolidine; the glidant is selected from the group consisting of talc, magnesium stearate, calcium stearate and calcium sulfate; and the C₁-C₃ alcohol is isopropanol.

7. A coated pesticidal agent obtainable by the process of claim 1 or 4, particularly a coated pesticidal agent which comprises
(A) a pesticidal agent core surrounded by
(B) a matrix comprising
(B-1) 2 to 25% by weight of a pH-dependent polymer selected from the group consisting of methacrylic acid and methyl methacrylate copolymers, maleic anhydride and styrene copolymers, and mixtures thereof;
(B-2) 0 to 5% by weight of a plasticizer;
(B-3) 5 to 45% by weight of an ultraviolet protector;
(B-4) 0 to 75% by weight of a stilbene compound;
(B-5) 0 to 10% by weight of a disintegrating agent; and
(B-6) 0% to 10% by weight of a glidant.

8. The coated pesticidal agent according to claim 7 wherein the ratio of the pesticidal agent to the matrix is about 1:1 to 1:10.

9. The coated pesticidal agent according to claim 7 wherein the pesticidal agent is an insecticidal pathogen; the plasticizer is selected from the group consisting of a polyethylene glycol, a polypropylene glycol, diethyl phthalate, dibutyl phthalate, a citric acid ester, castor oil and triacetin; the ultraviolet protector is selected from the group consisting of carbon black, a benzophenone, a dye and titanium dioxide; the disintegrating agent is selected from the group consisting of salts of the condensation products of formaldehyde with the sulfonation products of polycyclic aromatic compounds, a hydrophilic starch, carboxy methyl-cellulose and polyvinyl pyrrolidine; and the glidant is selected from the group consisting of talc, magnesium stearate, calcium stearate and calcium sulfate.

10. A wettable powder pesticidal composition which comprises
(i) 2 to 25% by weight of a wetting agent;
(ii) 2 to 40% by weight of a dispersing agent;
(iii) 10 to 70% by weight of a bulking agent;
(iv) 1 to 10% by weight of a flow enhancing agent;
(v) 0 to 20% by weight of a pH-modifying agent; and
(vi) 5 to 75% by weight of a coated pesticidal agent as defined in claim 7.

## Patentansprüche

1. Verfahren zur Herstellung eines beschichteten oder überzogenen pestiziden Mittels, wobei das Verfahren folgendes umfasst:
a) ein Bereitstellen einer wässerigen Mischung eines pH-abhängigen Polymers und gegebenenfalls eines Weichmachers;
b) ein Lösen des pH-abhängigen Polymers durch Einstellen des pH der Mischung von Schritt (a) auf einen pH, der über dem Solubilisations-pH des pH-abhängigen Polymers liegt;
c) ein Hinzugeben eines pestiziden Mittels, eines Schutzmittels gegen ultraviolette Strahlung, gegebenenfalls einer Stilben-Verbindung, gegebenenfalls eines Aufschlussmittels und gegebenenfalls eines Gleitmittels zu der Lösung von Schritt (b), und ein Mischen, um eine homogene Suspension herzustellen;
d) ein Trocknen der homogenen Suspension von Schritt (c); und gegebenenfalls
e) ein Mahlen des getrockneten Materials von Schritt (d).

2. Das Verfahren nach Anspruch 1, wobei das pH-abhängige Polymer ausgewählt ist aus der Gruppe, bestehend aus einer Methacrylsäure und Methylmethacrylat-Copolymer, einer Mischung aus Methacrylsäure und Methylmethacrylat-Copolymeren und einem Maleinsäureanhydrid und Styrol-Copolymer; der Weichmacher ausgewählt ist aus der Gruppe, bestehend aus einem Polyethylenglykol, einem Polypropylenglykol, Diethylphthalat, Dibutylphthalat, einem Zitronensäureester, Rizinusöl und Triacetin; die Base ausgewählt ist aus der Gruppe, bestehend aus Ammoniumhydroxid, einem Alkalimetallhydroxid und einem Erdalkalimetallhydroxid; das pestizide Mittel ein insektizides Pathogen ist; das Schutzmittel gegen ultraviolette Strahlung ausgewählt ist aus der Gruppe, bestehend aus Ruß, einem Benzophenon, einem Farbstoff und Titandioxid; der pH in Schritt (b) auf einen pH von 8.5 bis 10 eingestellt wird; das Aufschlussmittel ausgewählt ist aus der Gruppe, bestehend aus Salzen der Kondensationsprodukte von Formaldehyd mit den Sulfonierungsprodukten von polycyclischen aromatischen Verbindungen, einer hydrophilen Stärke, Carboxymethylcellulose und Polyvinylpyrrolidin; und das Gleitmittel ausgewählt ist aus der Gruppe, bestehend aus Talk, Magnesiumstearat, Calciumstearat und Calciumsulfat.

3. Das Verfahren nach Anspruch 2, wobei der Weichmacher ein Polyethylenglykol ist, das ein Molekulargewicht von etwa 300 bis 1.000 aufweist; die Base Ammoniumhydroxid ist; das insektizide Pathogen ein Virus ist, das ausgewählt ist aus der Gruppe, bestehend aus wildem Großen Schwammspinner-Kernpolyedervirus oder -NPV (Nuclear Polyhedrosis Virus), AcMNPV E2, AcMNPV L1, AcMNPV V8, V8vEGTDEL, VBvEGTDEL-AaIT und *Heliothis zea*-NPV; das Aufschlussmittel ein Natriumsulfonat eines Naphthalin-Formaldehyd-Kondensats ist; und das Gleitmittel Talk ist, und wobei das beschichtete pestizide Mittel eine Partikelgröße von weniger als etwa 20 µm aufweist.

4. Verfahren zur Herstellung eines beschichteten oder überzogenen pestiziden Mittels, wobei das Verfahren folgendes umfasst:
a) ein Mischen einer Mischung aus einem pH-abhängigen Polymer, einem pestiziden Mittel, gegebenenfalls einem Weichmachers, einem Schutzmittel gegen ultraviolette Strahlung, gegebenenfalls einer Stilben-Verbindung, gegebenenfalls einem Aufschlussmittel und gegebenenfalls einem Gleitmittel in einem organischen Lösungsmittel, das ausgewählt ist aus der Gruppe, bestehend aus Aceton, einem C₁-C₃-Alkohol und Mischungen davon;
b) ein Trocknen der homogenen Suspension von Schritt (a); und gegebenenfalls
c) ein Mahlen des getrockneten Materials von Schritt (b).

5. Das Verfahren nach Anspruch 4, wobei das organische Lösungsmittel eine Mischung aus Aceton und einem C₁-C₃-Alkohol ist, und das Verhältnis von Aceton zu dem C₁-C₃-Alkohol etwa 1:9 bis 9:1 beträgt.

6. Das Verfahren nach Anspruch 4, wobei das pH-abhängige Polymer ausgewählt ist aus der Gruppe, bestehend aus einer Methacrylsäure und Methylmethacrylat-Copolymer, einer Mischung aus Methacrylsäure und Methylmethacrylat-Copolymeren und einem Maleinsäureanhydrid und Styrol-Copolymer; das pestizide Mittel ein insektizides Pathogen ist; der Weichmacher ausgewählt ist aus der Gruppe, bestehend aus einem Polyethylenglykol, einem Polypropylenglykol, Diethylphthalat, Dibutylphthalat, einem Zitronensäureester, Rizinusöl und Triacetin; das Schutzmittel gegen ultraviolette Strahlung ausgewählt ist aus der Gruppe, bestehend aus Ruß, einem Benzophenon, einem Farbstoff und Titandioxid; das Aufschlussmittel ausgewählt ist aus der Gruppe, bestehend aus Salzen der Kondensationsprodukte von Formaldehyd mit den Sulfonierungsprodukten von polycyclischen aromatischen Verbindungen, einer hydrophilen Stärke, Carboxymethylcellulose und Polyvinylpyrrolidin; das Gleitmittel ausgewählt ist aus der Gruppe, bestehend aus Talk, Magnesiumstearat, Calciumstearat und Calciumsulfat; und der C₁-C₃-Alkohol Isopropanol ist.

7. Beschichtetes oder überzogenes pestizides Mittel, das erhältlich ist nach dem Verfahren von Anspruch 1 oder 4, insbesondere ein beschichtetes oder überzogenes pestizides Mittel, das folgendes umfasst
(A) einen Kern aus einem pestiziden Mittel, der umgeben ist von
(B) einer Matrix, umfassend
(B-1) 2 bis 25 Gewichts-% eines pH-abhängigen Polymers, das ausgewählt ist aus der Gruppe, bestehend aus Methacrylsäure und Methylmethacrylat-Copolymeren, Maleinsäureanhydrid und Styrol-Copolymeren und Mischungen davon;
(B-2) 0 bis 5 Gewichts-% eines Weichmachers;
(B-3) 5 bis 45 Gewichts-% eines Schutzmittels gegen ultraviolette Strahlung;
(B-4) 0 bis 75 Gewichts-% einer Stilben-Verbindung;
(B-5) 0 bis 10 Gewichts-% eines Aufschlussmittels; und
(B-6) 0 Gewichts-% bis 10 Gewichts-% eines Gleitmittels.

8. Das beschichtete oder überzogene pestizide Mittel gemäß Anspruch 7, wobei das Verhältnis des pestiziden Mittels zu der Matrix etwa 1:1 bis 1:10 beträgt.

9. Das beschichtete oder überzogene pestizide Mittel gemäß Anspruch 7, wobei das pestizide Mittel ein insektizides Pathogen ist; der Weichmacher ausgewählt ist aus der Gruppe, bestehend aus einem Polyethylenglykol, einem Polypropylenglykol, Diethylphthalat, Dibutylphthalat, einem Zitronensäureester, Rizinusöl und Triacetin; das Schutzmittel gegen ultraviolette Strahlung ausgewählt ist aus der Gruppe, bestehend aus Ruß, einem Benzophenon, einem Farbstoff und Titandioxid; das Aufschlussmittel ausgewählt ist aus der Gruppe, bestehend aus Salzen der Kondensationsprodukte von Formaldehyd mit den Sulfonierungsprodukten von polycyclischen aromatischen Verbindungen, einer hydrophilen Stärke, Carboxymethylcellulose und Polyvinylpyrrolidin; und das Gleitmittel ausgewählt ist aus der Gruppe, bestehend aus Talk, Magnesiumstearat, Calciumstearat und Calciumsulfat.

10. Benetzbare Pulver-Pestizidzusammensetzung, die folgendes umfasst:
(i) 2 bis 25 Gewichts-% eines Benetzungsmittels;
(ii) 2 bis 40 Gewichts-% eines Dispergiermittels;
(iii) 10 bis 70 Gewichts-% eines Grund- oder Schüttgut- oder Füllmaterials;
(iv) 1 bis 10 Gewichts-% eines Fließverbesserers oder Verlaufsmittels oder eines die Fließfähigkeit steigernden Mittels;
(v) 0 bis 20 Gewichts-% eines den pH modifizierenden Mittels; und
(vi) 5 bis 75 Gewichts-% eines beschichteten oder überzogenen pestiziden Mittels, wie es in Anspruch 7 definiert ist.

## Revendications

1. Procédé pour la préparation d'un agent pesticide enrobé, qui comprend les étapes suivantes :
a) préparer un mélange aqueux d'un polymère dépendant du pH et facultativement d'un plastifiant ;
b) dissoudre le polymère dépendant du pH en ajustant le pH du mélange de l'étape (a) à un pH supérieur au pH de solubilisation du polymère dépendant du pH ;
c) ajouter un agent pesticide, un agent protecteur anti-ultraviolet, facultativement un composé du stilbène, facultativement un agent désintégrant et facultativement un agent d'écoulement à la solution de l'étape (b), et mélanger pour produire une suspension homogène ;
d) sécher la suspension homogène de l'étape (c) ; et facultativement
e) broyer la matière séchée de l'étape (d).

2. Procédé selon la revendication 1, dans lequel le polymère dépendant du pH est choisi dans le groupe formé par un copolymère d'acide méthacrylique et de méthacrylate de méthyle, un mélange de copolymères d'acide méthacrylique et de méthacrylate de méthyle et un copolymère d'anhydride maléique et de styrène ; le plastifiant est choisi dans le groupe formé par un polyéthylène-glycol, un polypropylène-glycol, le phtalate de diéthyle, le phtalate de dibutyle, un ester d'acide citrique, l'huile de ricin et la triacétine ; la base est choisie dans le groupe formé par l'hydroxyde d'ammonium, un hydroxyde de métal alcalin et un hydroxyde de métal alcalino-terreux ; l'agent pesticide est un agent pathogène insecticide ; l'agent protecteur anti-ultraviolet est choisi dans le groupe formé par le noir de carbone, une benzophénone, un colorant et le bioxyde de titane ; le pH est ajusté dans l'étape (b) à un pH de 8,5 à 10 ; l'agent désintégrant est choisi dans le groupe formé par les sels des produits de condensation de formaldéhyde avec les produits de sulfonation de composés aromatiques polycycliques, un amidon hydrophile, la carboxyméthylcellulose et la polyvinylpyrrolidine ; et l'agent d'écoulement est choisi dans le groupe formé par le talc, le stéarate de magnésium, le stéarate de calcium et le sulfate de calcium.

3. Procédé selon la revendication 2, dans lequel le plastifiant est un polyéthylène-glycol ayant un poids moléculaire d'environ 300 à 1000 ; la base est l'hydroxyde d'ammonium ; l'agent pathogène insecticide est un virus choisi dans le groupe formé par le NPV du bombyx disparate sauvage, AcMNPV E2, AcMNPV L1, AcMNPV V8, V8vEGTDEL, V8vEGTDEL-AaIT et le NPV de *Heliothis zea* ; l'agent désintégrant est un sulfonate de sodium d'un produit de condensation naphtalène-formaldéhyde ; et l'agent d'écoulement est le talc, et dans lequel l'agent pesticide enrobé a une taille de particules inférieure à environ 20 µm.

4. Procédé pour la préparation d'un agent pesticide enrobé, qui comprend les étapes suivantes :
a) mélanger un mélange d'un polymère dépendant du pH, d'un agent pesticide, facultativement d'un plastifiant, d'un agent protecteur anti-ultraviolet, facultativement d'un composé du stilbène, facultativement d'un agent désintégrant et facultativement d'un agent d'écoulement dans un solvant organique choisi dans le groupe formé par l'acétone, un alcool en C₁-C₃, et leurs mélanges ;
b) sécher la suspension homogène de l'étape (a) ; et facultativement
c) broyer la matière séchée de l'étape (b).

5. Procédé selon la revendication 4, dans lequel le solvant organique est un mélange d'acétone et d'un alcool en C₁-C₃ et le rapport de l'acétone à l'alcool en C₁-C₃ est d'environ 1:9 à 9:1.

6. Procédé selon la revendication 4, dans lequel le polymère dépendant du pH est choisi dans le groupe formé par un copolymère d'acide méthacrylique et de méthacrylate de méthyle, un mélange de copolymères d'acide méthacrylique et de méthacrylate de méthyle et un copolymère d'anhydride maléique et de styrène ; l'agent pesticide est un agent pathogène insecticide ; le plastifiant est choisi dans le groupe formé par un polyéthylène-glycol, un polypropylène-glycol, le phtalate de diéthyle, le phtalate de dibutyle, un ester d'acide citrique, l'huile de ricin et la triacétine ; l'agent protecteur anti-ultraviolet est choisi dans le groupe formé par le noir de carbone, une benzophénone, un colorant et le bioxyde de titane ; l'agent désintégrant est choisi dans le groupe formé par les sels des produits de condensation de formaldéhyde avec les produits de sulfonation de composés aromatiques polycycliques, un amidon hydrophile, la carboxyméthylcellulose et la polyvinylpyrrolidine ; l'agent d'écoulement est choisi dans le groupe formé par le talc, le stéarate de magnésium, le stéarate de calcium et le sulfate de calcium ; et l'alcool en C₁-C₃ est l'isopropanol.

7. Agent pesticide enrobé pouvant être obtenu par le procédé de la revendication 1 ou 4, en particulier un agent pesticide enrobé qui comprend
(A) un noyau d'agent pesticide entouré par
(B) une matrice comprenant
(B-1) 2 à 25 % en poids d'un polymère dépendant du pH choisi dans le groupe formé par des copolymères d'acide méthacrylique et de méthacrylate de méthyle, des copolymères d'anhydride maléique et de styrène, et leurs mélanges ;
(B-2) 0 à 5 % en poids d'un plastifiant ;
(B-3) 5 à 45 % en poids d'un agent protecteur anti-ultraviolet ;
(B-4) 0 à 75 % en poids d'un composé du stilbène ;
(B-5) 0 à 10 % en poids d'un agent désintégrant ; et
(B-6) 0 % à 10 % en poids d'un agent d'écoulement.

8. Agent pesticide enrobé selon la revendication 7, dans lequel le rapport de l'agent pesticide à la matrice est d'environ 1:1 à 1:10.

9. Agent pesticide enrobé selon la revendication 7, dans lequel l'agent pesticide est un agent insecticide pathogène ; le plastifiant est choisi dans le groupe formé par un polyéthylène-glycol, un polypropylène-glycol, le phtalate de diéthyle, le phtalate de dibutyle, un ester d'acide citrique, l'huile de ricin et la triacétine ; l'agent protecteur anti-ultraviolet est choisi dans le groupe formé par le noir de carbone, une benzophénone, un colorant et le bioxyde de titane ; l'agent désintégrant est choisi dans le groupe formé par les sels des produits de condensation de formaldéhyde avec les produits de sulfonation de composés aromatiques polycycliques, un amidon hydrophile, la carboxyméthylcellulose et la polyvinylpyrrolidine ; et l'agent d'écoulement est choisi dans le groupe formé par le talc, le stéarate de magnésium, le stéarate de calcium et le sulfate de calcium.

10. Composition pesticide en poudre mouillable, qui comprend
(i) 2 à 25 % en poids d'un agent mouillant ;
(ii) 2 à 40 % en poids d'un agent dispersant ;
(iii) 10 à 70 % en poids d'une charge diluante ;
(iv) 1 à 10 % en poids d'un agent améliorant l'écoulement ;
(v) 0 à 20 % en poids d'un agent modificateur de pH ; et
(vi) 5 à 75 % en poids d'un agent pesticide enrobé tel que défini dans la revendication 7.
